Europäisches Patentamt

European Patent Office

Office européen des brevets

(11) Publication number: **0 027 361**
**A1**

## EUROPEAN PATENT APPLICATION

(21) Application number: **80303571.6**

(22) Date of filing: **10.10.80**

(51) Int. Cl.³: **A 61 B 1/06**
**F 21 V 25/10**

(30) Priority: **11.10.79 JP 140583/79 U**

. (43) date of publication of application:
**22.04.81 Bulletin 81/16**

(84) Designated Contracting States:
**AT BE CH DE FR GB IT LI NL SE**

(71) Applicant: **OLYMPUS OPTICAL CO., LTD.**
**43-2, 2-chome, Hatagaya Shibuya-ku**
**Tokyo(JP)**

(72) Inventor: **Tsukaya, Takashi**
**4-22-13, Owada-Cho**
**Hachioji-Shi Tokyo(JP)**

(74) Representative: **Jones, Colin et al,**
**W.P. THOMPSON & CO. Coopers Building Church Street**
**Liverpool L1 3AB(GB)**

(54) Light source assembly for endoscope.

(57) A light source assembly (1) for an endoscope (4) includes a heat-sensitive circuit breaker (11) which operates to turn a power supply circuit (12b) off to de-energize an illumination lamp (10), by detecting a rapid temperature rise in the assembly which may be caused whenever a cooling fan (9) is inoperable or ventilation holes (2,3) are blocked by adjacent objects or wall.

EP 0 027 361 A1

Croydon Printing Company Ltd.

1.

## DESCRIPTION

"LIGHT SOURCE ASSEMBLY FOR ENDOSCOPE"

The invention relates to a light source assembly for an endoscope.

In the conventional practice, an affected part is observed by the use of an endoscope which is inserted into the coeliac cavity. Light flux from a lamp of a light source assembly associated with the endoscope is conducted along a light guide, which is received within the endoscope, and illuminates the part to be observed. The illuminated affected part is observed through an image guide and an eyepiece assembly on the endoscope.

Since the lamp used has an increased wattage, the collection of light flux from the lamp onto the input end face of the light guide results in generating a large quantity of heat. To accommodate for this problem, the light source assembly is internally air cooled, usually by the provision of a cooling fan therein which draws in external air through ventilation holes for circulation therein. However, the light source assembly is normally or frequently located close to or against the wall of a room for the consideration of space requirements. This location may cause the ventilation holes to be blocked by adjacent objects, preventing a satisfactory air induction and circulation from being achieved. Consequently, the cooling efficiency may be degraded to cause a temperature rise of the interior of the assembly, whereby the internal components such as the lamp, the end face of the light guide, power transformer, lead wires or even the cooling fan may be burnt out or have their useful life substantially reduced.

If the lead wires associated with the cooling fan are broken for some reason or the power supply of the fan fails, the cooling fan is inoperable, again preventing a satisfactory air admission and circulation to cause a substantial temperature rise, giving rise to the described disadvantages such as burn-out.

It is an object of the invention to eliminate above disadvantages by providing a light source assembly for an endoscope including a heat sensitive interruptor which operates to turn a power supply circuit off in response to an abnormal temperature rise sensed therein whenever a cooling fan is inoperable or ventilation holes used for inducting air into the assembly are blocked by a wall or other object.

In accordance with the invention, if the internal temperature of the assembly rises abnormally high, the heat sensitive interruptor detects it to turn a power supply circuit off, thus interrupting the power supply to the illumination lamp. In this manner, a further temperature rise is avoided, which might otherwise cause the components of the assembly such as power transformer, the end face of a light guide, illumination lamp, lead wires and cooling fan to be burnt out.

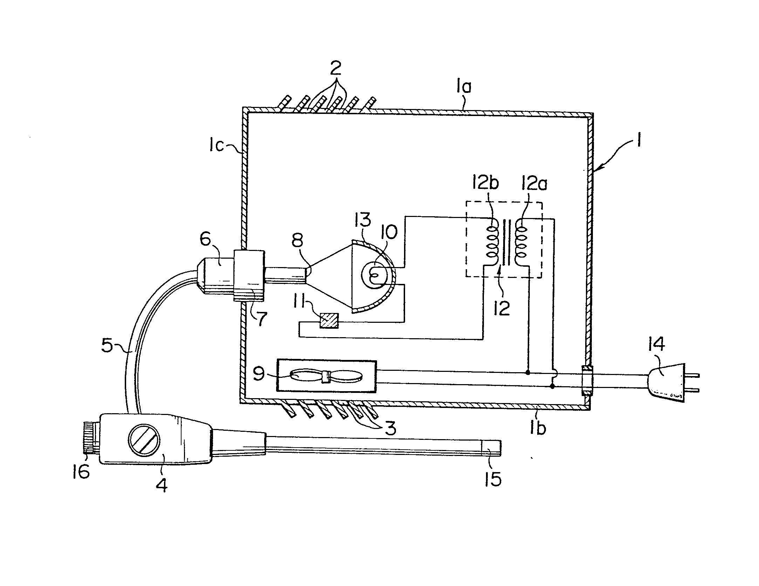

The invention is further described, by way of example, with reference to the drawing which is a schematic view of a light source assembly for an endoscope according to one embodiment of the invention.

Referring to the drawing, a light source assembly 1 for an endoscope 4 includes a housing 1 having a pair of oppositely located sidewalls 1a, 1b in which ventilation holes 2,3, respectively, are formed. The ventilation holes 2 are used to admit air into the assembly while the ventilation holes 3 are used to exhaust hot air. The housing 1 also includes a front wall 1c having a socket 7 mounted therein for connection with a connector 6, which is provided on the free end of an extension 5 from the endoscope 4. A light guide is disposed inside the extension 5, and leads into the housing 1, presenting an end face 8 therein. A cooling fan 9 is located inside the housing 1 adjacent to the exhaust holes 3 for expelling hot air produced within the housing 1 therethrough. An illumination lamp 10 is disposed in opposing relationship with the end face 8 of the light guide, and a heat-sensitive circuit breaker 11 is located below a path between the end face 8 and the lamp 10. The cooling fan 9 is connected to a plug 14, which is adapted to be inserted into a convenient outlet of a commercial power supply. A power transformer 12 has its primary winding 12a connected to the commercial supply through the plug 14 and has its secondary winding 12b connected in series with the filament of the lamp 10 and the breaker 11. The breaker 11 may comprise a bimetal switch, for example. The lamp 10 is associated with a lamp cowl 13, which functions to collect light and direct it onto the end face 8 of the light guide. The light guide may comprise a bundle of optical fibres which is disposed to extend through the interior of the extension 5 and the endoscope 4. Thus the light collected on the end face 8 passes through the bundle to be directed out of the distal end 15 of the endoscope 4 to illuminate an affected part within the coeliac cavity. The light image of the affected part thus illuminated is focussed by an objective lens, not shown, and transmitted through an image guide, not shown, of the endoscope 4 for observation through an

4.

eyepiece assembly 16.

In operation, if the air admission holes 2 are blocked by adjacent objects, or the exhaust holes 3 are placed against a wall, or the cooling fan 9 is inoperable as a result of breakage of its lead wires, for example, the air admission and circulation through the light source assembly are degraded to cause a rapid temperature rise, thus substantially reducing the cooling efficiency. This temperature rise is detected by the heat sensitive circuit breaker 11, which operates to interrupt the connection between the lamp 10 and the power transformer 12 to de-energize the lamp 10. The lamp 10 then ceases to emit light, and a further temperature rise is avoided.

In this manner, the heat-sensitive breaker 11 operates to interrupt the power supply to the lamp 10 in the event the internal temperature of the assembly rises abnormally high. Accordingly, lamp 10, end face 8, power transformer 12, lead wires and cooling fan 9 cannot be damaged or burnt out as the internal temperature rises abnormally high. It is to be understood that the location of the circuit breaker 11 is not limited to the specific location shown and described above, but may be anywhere close to the lamp 10 and the end face 8. Alternatively, a plurality of circuit breakers may be disposed close to individual one of them.

5.

## CLAIMS

1. A light source assembly (1) for an endoscope (4) including a light source (10) for producing light which is introduced into an illumination optical system of the endoscope for transmission therein to illuminate an object to be observed with the endoscope; characterized by the provision of means (11) for detecting an abnormal temperature rise which may be caused by heat produced by the light source (10).

2. A light source assembly according to claim 1, in which said means (11) comprises a heat-sensitive circuit breaker connected in series with a power supply circuit (12) of the light source (10).

3. A light source assembly according to claim 2, in which the heat-sensitive circuit breaker (11) comprises a bimetal switch.

European Patent Office

**EUROPEAN SEARCH REPORT**

Application number

EP 80 30 3571

| Category | Citation of document with indication, where appropriate, of relevant passages | Relevant to claim |
|---|---|---|
| X | FR - A - 2 305 092 (DENTSPLY INTERNATIONAL, INC.)<br><br>* Page 4, lines 22-33; page 7, line 30 page 8, line 8; page 13, line 19 - page 14, line 18; page 15, line 23 - page 16, line 4; figures 1-3,5 *<br><br>-- | 1,2 |
| X | US - A - 3 712 984 (O.E. LIENHARD/ CONRAD PRECISION INDUSTRIES, INC.)<br><br>* Column 1, lines 12-25; column 6, lines 1-21; column 7, lines 24-38; figures 3,8 *<br><br>-- | 1,2 |
| | FR - A - 1 505 636 (TEXAS INSTRUMENTS INC.)<br><br>* Page 1, left-hand column, lines 1-7; page 1, right-hand column, lines 1-31; page 2, left-hand column, lines 17-39; page 2, right-hand column, line 33 - page 3, left-hand column, line 9; page 3, right-hand column, line 36 - page 4, left-hand column, line 18; figures 1,3 *<br><br>-- | 1,2 |
| | DE - C - 842 751 (E. BAUER GmbH)<br><br>* Page 1, lines 1-8; page 2, lines 16-73; figures 1,2 *<br><br>-- | 1-3 |
| | US - A - 3 999 099 (H. HERION)<br><br>* Abstract; column 2, lines 12-48; column 3, line 42 - column 4, line 24; figures 1-3 *<br><br>---- | 2,3 |

**DOCUMENTS CONSIDERED TO BE RELEVANT**

**CLASSIFICATION OF THE APPLICATION (Int. Cl.³)**

A 61 B 1/06
F 21 V 25/10

**TECHNICAL FIELDS SEARCHED (Int. Cl.³)**

A 61 B 1/06
F 21 V 25/10
29/00
F 21 S 5/00
H 05 B 39/10
41/46
G 03 B 21/16

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 16-01-1981 | RIEB |

EPO Form 1503.1 06.78